# EUROPEAN PATENT APPLICATION

(11) **EP 3 936 601 A1**
(43) Date of publication of application: **12.01.2022**
(21) Application number: 20184330.7
(22) Date of filing: 06.07.2020
(51) Int. Cl.: C12M 1/26

(54) **CLARIFICATION SETUP OF A BIOPROCESSING INSTALLATION**

(71) Applicant: Sartorius Stedim Biotech GmbH, 37079 Göttingen (DE)
(72) Inventor: SABALLUS, Martin, 44149 Dortmund (DE)
(74) Representative: Gottschald Patentanwälte Partnerschaft mbB

(57) **Abstract**

The invention is directed to a clarification setup of a bioprocessing installation for the clarification of a cell broth by centrifugation and subsequent filtration, wherein, for the centrifugation, the clarification setup comprises a centrifuge (1), wherein the centrifuge (1) comprises at least one centrifuge chamber (2) with a chamber inlet (3) and a chamber outlet (4), wherein the clarification setup comprises a liquid pumping arrangement (5) assigned to the centrifuge (1) and a liquid network (6) with a number of liquid lines (7) communicating with the liquid pumping arrangement (5), wherein the clarification setup comprises a process control (8) for controlling at least the centrifuge (1) and the liquid pumping arrangement (5). It is proposed that for filtration, the clarification setup comprises a filter arrangement (9), that the liquid network (6) provides an outlet supernatant line (10) for a liquid connection between the chamber outlet (4) and the filter arrangement (9), which is at least temporarily, preferably permanently, liquid-tight, such that due to this liquid-tight condition of the outlet supernatant line (10) the liquid pumping arrangement (5) as such may drive the supernatant from the chamber outlet (4) to the filter arrangement (9) via the outlet supernatant line (10).

## Description

The present invention relates to a clarification setup of a bioprocessing installation according to the general portion of claim 1, a bioprocess installation with such a clarification setup according to claim 15 and a method for operating such a clarification setup according to claim 16.

The bioprocessing installation in question may be applied in various fields of bioprocessing technology. High efficiency in this field has been driven by the increasing demand for biopharmaceutical drugs. The efficiency in this sense is regarding not only the cost-effectiveness of the components to be used, but also the controllability of the processes connected thereto.

The clarification setup in question serves for the clarification of a cell broth by centrifugation and subsequent filtration. The cell broth is to be understood as a suspension comprising a liquid portion including supernatant comprising soluble (bio)molecules including the product of interest and comprising a solid portion comprising cells, cell debris and other solid particles.

The clarification serves to gain the supernatant including the product of interest from the cell broth by separating solid particles such as cells and cell debris.

The known clarification setup (EP 2 310 486 B1), which is the starting point for the invention, comprises a centrifuge with a number of centrifuge chambers, which are each assigned a chamber inlet and a chamber outlet. The clarification setup also comprises a liquid pumping arrangement assigned to the centrifuge and a liquid network for the transport of the liquid, which is based on the cell broth to be clarified. Finally, the clarification setup comprises a process control for controlling at least the centrifuge and the liquid pumping arrangement.

It is also known that, depending on the application, it is necessary to filter the centrifuge solution by a filter arrangement comprising, for example, a depth filter and a sterile filter ("examining single use harvest clarification options: a case study comparing depth-filter turbidities and recoveries", Manish K. Sharma, Bioprocess International, 2017).

The known clarification setup as such is running in a highly efficient manner. The same is to be said for the known filter arrangements that may be used to filter solid particles from the centrifuged liquid, called the supernatant. However, the resulting combination is of restricted efficiency in view of redundancies in the mechanical setup as well as in the controllability of the overall process.

It is therefore the object of the invention, to improve the known clarification setup such that its combined operation with a subsequent filter arrangement is of increased efficiency.

The above noted problem is solved by a clarification setup according to the general portion of claim 1 with the features of the characterizing portion of claim 1.

The general concept underlying the invention is based on a liquid tight connection between the chamber outlet and the filter arrangement. First of all, this allows for the operation without any intermediate reservoir for the liquid between the chamber outlet and the filter arrangement. All effects with such an intermediate reservoir, which has proven to lead to undesired drawbacks, are eliminated. Second of all, the liquid pumping arrangement, which is assigned to the centrifuge, may well be used to pump the centrifuged liquid to and through the filter arrangement. This means, that the filter arrangement as such does not need a separate liquid pumping arrangement, which is not only cost efficient, but is also making the control of the overall process easier and more efficient. Furthermore, the absence of an intermediate reservoir and a separate liquid pumping arrangement for the filter arrangement significantly reduces the footprint of the clarification set-up which is especially beneficial with respect to large-scale applications in the pharmaceutical industry and limited space to run process facilities. Finally, due to the proposed closed outlet supernatant line, no fluid may come into contact with any component outside this closed system, which leads to a reduced risk of contamination and a particularly environment friendly process and increased process savety.

In detail, it is proposed, that for filtration, the clarification setup comprises a filter arrangement, that the liquid networks provides an outlet supernatant line for a liquid connection between the chamber outlet and the filter arrangement, which is at least temporarily, preferably permanently, liquid-tight, such that due to this liquid-tight condition the liquid pumping arrangement as such may drive the supernatant from the chamber outlet to the filter arrangement via the outlet supernatant line.

The preferred embodiment according to claim 2 regards the combination of components in a self contained unit. The expression "self contained unit" represents a unit that is physically one single unit and that as a unit provides a function, that results from the combination of its components. With a structure according to claim 2, the realization of costly redundant components such as separate liquid pumping units for the centrifuge and the filter arrangement, may be prevented.

The process control is preferably designed to execute a number of predetermined process cycles according to claim 3, such as a loading cycle, a washing cycle and a discharging cycle. Such cycles are preferably each defined by a software module, which may be run on the process control.

Claims 4 to 6 are directed to the realization of the above noted, most essential process cycles, namely the loading cycle, the washing cycle and the discharging cycle. Due to the liquid tight condition of the outlet supernatant line, the liquid pressure and/or the liquid flow may easily be controlled by the liquid pumping arrangement.

The preferred embodiment according to claim 7 is directed to providing a valve arrangement for activating/deactivating the liquid lines of the liquid network in order to support the execution of the respective cycle. Here, deactivating means, that liquid flow through the respective liquid line may be blocked by the respective valve. Activating means, accordingly, that liquid flow is allowed through the respective liquid line.

According to claim 8, activating and deactivating of the filter arrangement or at least one filter of the filter arrangement is possible by the control of the valve arrangement. This allows for example a proper reaction to the blockage of a filter of the filter arrangement. In addition according to claim 9, the valve arrangement may be used for the redirection of liquid, for example, in order to flush the filter arrangement. This makes it possible to double use certain liquid lines for different tasks.

Claim 10 clarifies, that the filter arrangement may comprise at least one filter stage, peferably two filter stages comprising a depth filter stage and a steril filter stage, which may be individually laid out. The proposed clarification setup allows to realize a nearly unrestricted range of filter setups. Due to the centralized process control, it is possible to directly react to a filter blockage, for example by terminating the execution of the respective process cycle.

Claims 11 to 13 are directed to a sensor arrangement for measuring properties of the liquid in at least one of the liquid lines. Such properties may be the occurrence level of biomass and/or supernatant, the liquid pressure, the liquid flow or the like. The resulting sensor signals are being provided to the process control, which allows automation of the process cycles to a wide extent.

The term "occurrence level" in this application generally is a variable, which represents the degree of occurrence of the respective entity, here and preferably of biomass and/or supernatant, in the respective liquid line. This variable may represent a continuous range between "occurrence" and "no occurrence". This is for example the concentration of the respective entity within the liquid in the liquid line. The occurrence level may also represent a binary information being either "occurrence" or "no occurrence" of the entity in the liquid line.

In order to guarantee a cost efficient process and at the same time perfectly clean condition of the clarification setup, according to claim 14, at least part of the clarification setup is provided as a single use component.

A second independent teaching according to claim 15 is directed to a bioprocess installation with a proposed clarification setup and with a cell broth source in the form of a storage vessel or a production vessel, such as a bioreactor. All explanations given with regard to the first teaching are fully applicable to this second teaching.

A third independent teaching according to claim 16 is directed to a method for operating a proposed clarification setup, wherein the clarification setup comprises a valve arrangement controlled by the process control and wherein at least one filter of the filter arrangement is being deactivated and activated by shutting and unshutting the respective filter. Again, all explanations given with regard to the first teaching are fully applicable to this third teaching.

Preferably, the proposed method is based on a sensor arrangement for measuring properties of the liquid in at least one of the liquid lines, which sensor arrangement provides sensor signals to the process control and that at least one filter of the filter arrangement is being deactivated/activated based on the sensor signals of the sensor arrangement. Here as well, all explanations given with regard to the first teaching, in particular in view of the application of a sensor arrangement, are fully applicable.

In the following, a preferred embodiment of the invention is being described with regard to the drawings. In the drawings show
- Fig. 1: a schematic view on a proposed clarification setup during the loading cycle,
- Fig. 2: a schematic view on the clarification setup according to fig. 1 during the washing cycle and
- Fig. 3: a schematic view on the clarification setup according to fig. 1 during the discharging cycle.

The proposed clarification setup, here and preferably, is assigned to the downstream process of a cell culture containing a storage vessel or a production vessel such as a bioreactor, processing a liquid in the form of a cell broth. Accordingly, the wording "liquid" is to be understood in a broad sense. It includes not only liquids as such, but also solutions and suspensions with particles like cells, cell debris, etc.

This clarification setup may be operated in different operating modes. A first operating mode, which is the focus of the present invention, is the clarification of the cell broth from any solid particles by centrifugation and subsequent filtration. The goal here is to separate the cell broth from solid particles such as cells, cell debris, etc., which solid particles are considered biomass in the following. The product to be obtained in this first operating mode is the supernatant of the cell broth containing a product of interest, e.g. a recombinant protein such as an antibody.

A second operating mode, which may generally be realized by the present invention as well, is the clarification of cells in the cell broth from supernatant. The product to be obtained in this second operating mode are the cells in the cell broth.

For the centrifugation, the clarification setup comprises a centrifuge 1, wherein the centrifuge 1 comprises at least one centrifuge chamber 2.1, 2.2, 2.3, 2.4, each with a chamber inlet 3 and a chamber outlet 4. In the drawings, the altogether four centrifuge chambers 2.1, 2.2, 2.3, 2.4 are indicated. In this particular case, the chamber inlets 3 of the centrifuge chambers 2.1, 2.2, 2.3, 2.4 are connected with each other and the chamber outlets 4 of the centrifuge chambers 2.1, 2.2, 2.3, 2.4 are connected with each other to act as a single chamber inlet respective a single chamber outlet from the outside.

Here and preferably, each chamber 2.1, 2.2, 2.3, 2.4 is located offset a centrifuge rotor axis, wherein, further preferably, the respective chamber inlet 3 is located further away from the centrifuge rotor axis than the respective chamber outlet 4.

The expression "chamber inlet" means that the liquid to be centrifuged enters the respective chamber 2.1, 2.2, 2.3, 2.4 via the respective chamber inlet 3. The expression "chamber outlet" means that the centrifuged liquid exits the respective chamber 2.1, 2.2, 2.3, 2.4 via the respective chamber outlet 4. This is only to be understood as a definition of the fluid interface of the respective chamber 2.1, 2.2, 2.3, 2.4. As will be explained later, in certain situations, the chamber inlets 3 may be used as outlets and the chamber outlets 4 may be used as inlets respectively.

In order to facilitate the explanation of the invention, in the following, the specification states only one centrifuge chamber 2.1 with a chamber inlet 3 and a chamber outlet 4. All explanations regarding this centrifuge chamber 2.1 are fully applicable to any other centrifuge chamber, which may be provided.

The clarification setup comprises a liquid pumping arrangement 5 assigned to the centrifuge 1 and a liquid network 6 with a number of liquid lines 7 communicating with the pumping arrangement 5, wherein the clarification setup comprises a process control 8 for controlling at least the centrifuge 1 and the liquid pumping arrangement 5. Here and preferably, the pumping arrangement 5 is located upstream the centrifuge chamber 2.1 during the loading cycle to be explained.

The process control 8 may be realized as a central unit controlling all or at least most of the components of the clarification setup. The process control 8 may also be realized in a decentralized structure, comprising a number of decentralized units. In any case, the process control 8 comprises at least one microprocessor, on which a software may be run.

Here and preferably, the centrifuge 1 is designed as a fluidized bed centrifuge for performing a continuous centrifugation process. The preferred setup of the centrifuge 1 is described in European Patent EP 2 485 846 B1.

The centrifuge 1 comprises a rotor, which may be rotated around the centrifuge rotor axis by a preferably electric motor, which is controlled by the process control 8 for the realization of a centrifuge revolution speed. For centrifugation, the liquid pumping arrangement 5 pumps cell broth through the centrifuge chamber 2.1.
The centrifuge revolution speed as well as the pumping rate are adjusted, preferably by the process control 8, with the aim to establish a fluidized bed of particles such as cells or cell debris in the centrifuge chamber 2.1. A fluidized bed is achieved when the centrifugal force on a particle is equal to the opposing fluid flow force so that a zero net force is exerted on the particle.

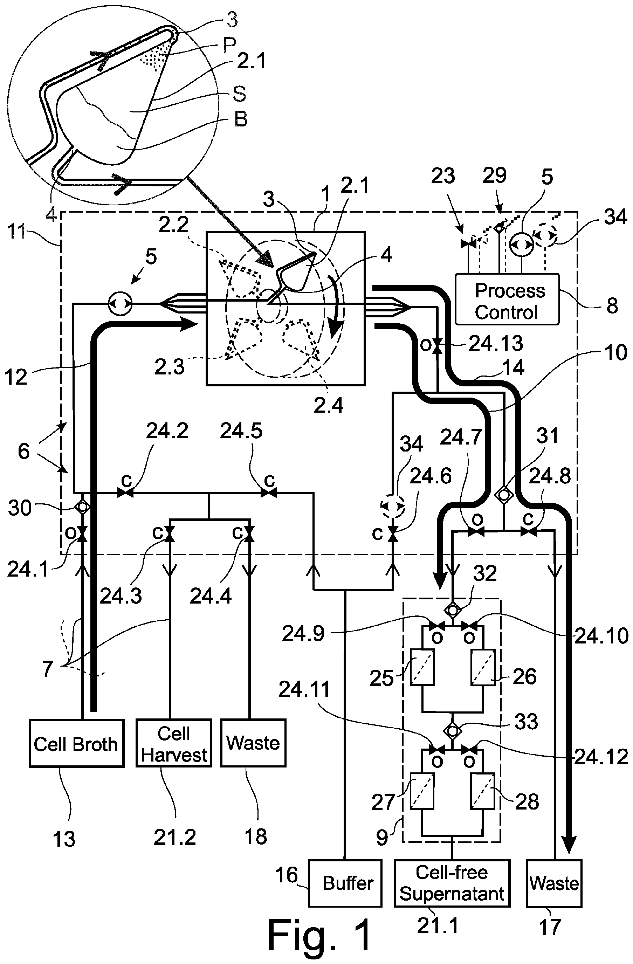

In order to obtain a particle free supernatant, the centrifuged cell broth is pumped through a filter arrangement 9 of the clarification setup as shown in Fig. 1. For this, the liquid network 6 provides an outlet supernatant line 10 for a liquid connection between the chamber outlet 4 and the filter arrangement 9. Downstream of the filter arrangement 9 is provided a supernatant reception 21.1, in particular a supernatant vessel.

For better understanding, in the detained view of the centrifuge chamber 2.1 in Fig. 1, the buffer is indicated with reference "B", the supernatant is indicated with reference "S" and the particles are indicated with reference "P".

It is particularly essential for the invention, that the outlet supernatant line 10 is at least temporarily, preferably permanently, liquid-tight, such that due to this liquid-tight condition of the outlet supernatant line 10 the liquid pumping arrangement 5 as such may drive the supernatant from the chamber outlet 4 to the filter arrangement 9 via the outlet supernatant line 10. In other words, the outlet supernatant line 10 is at least temporarily, preferably permanently, liquid tight such that the liquid pressure and/or the liquid flow may be controlled by the liquid pumping arrangement 5, which is assigned to the centrifuge 1. The expression "temporarily" means, that in the installed state of the clarification setup, the liquid tightness may be canceled for a certain time, for example by switching of a valve or the like. The expression "permanently" means, that in the installed state of the clarification setup, the liquid tightness is always guaranteed. The later embodiment includes, preferably, the outlet supernatant line 10 being fluid tight also in the uninstalled state.

As a result, the liquid connection between the centrifuge 1 and the filter arrangement 9 via the outlet supernatant line 10 at least temporarily, preferably permanently, is a closed system regarding liquids in the above noted sense. In addition, preferably, the connection between the centrifuge 1 and the filter arrangement 9 via the outlet supernatant line 10 at least temporarily, preferably permanently, is a closed system regarding gaseous media.

Here and preferably, the centrifuge 1 and the liquid pumping arrangement 5 are combined in a unit 11. Preferably, this means that a unit carrier is provided, which carries all unit components, here the centrifuge 1 and the liquid pumping arrangement 5. The unit carrier may well include a housing, which houses all unit components.

The above noted unit 11 is preferably a self contained unit. The expression "self contained unit" means, that this unit 11 includes all components to provide a desired function, here the function of centrifugation. Such a self contained unit provides interfaces in mechanical, electrical, electronical and liquidical view, in order to be chained into an overall process.

Further preferably, the filter arrangement 9 is part of the above noted unit 11. As an alternative or in addition, the process control 8 is part of this self-contained unit 11 as well. As a result, the part of the downstream process including the centrifugation and subsequent filtration may well be realized as a fully integrated process in a single, preferably self contained, unit.

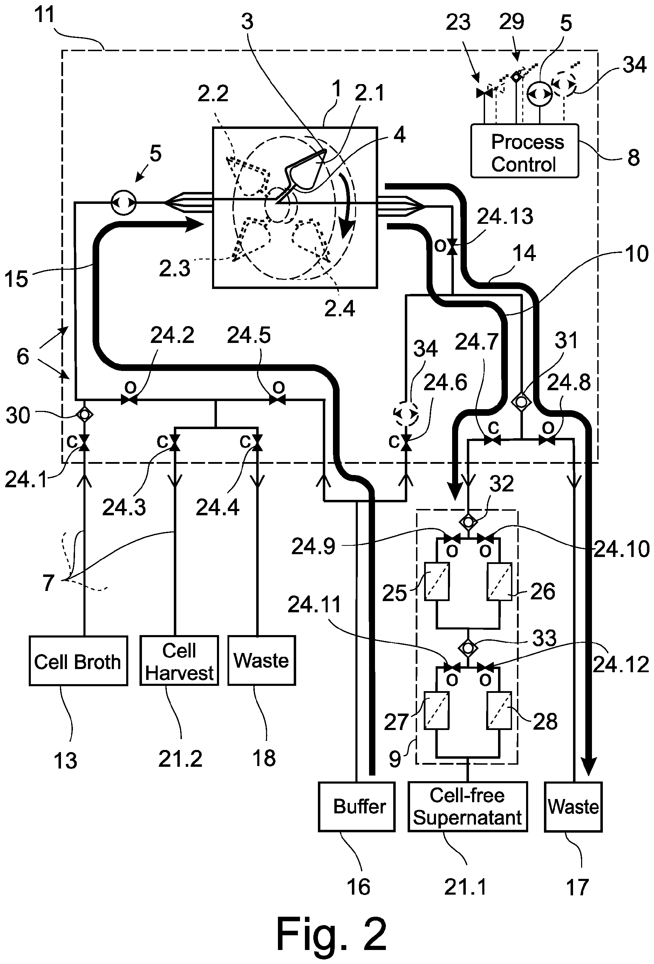

The process control 8 is preferably designed to periodically execute a number of predetermined process cycles sequentially by controlling at least the centrifuge 1 and the liquid pumping arrangement 5. One of the process cycles is a loading cycle as indicated in Fig. 1, during which cell broth is pumped into the chamber inlet 3. Another one of the cycles may be a washing cycle as indicated in Fig. 2, during which a buffer is pumped into the chamber inlet 3. Finally, another one of the cycles may be a discharging cycle, during which a buffer is pumped to the chamber outlet 4. While those cycles of loading cycle, washing cycle and discharging cycle are well known for fluidized bed centrifugation, it is interesting here that the process control 8 is automatically executing those cycles sequentially, according to a certain control strategy. In the easiest case, the control strategy includes the execution of the respective cycles according to a fixed sequence in a fixed time pattern. However, the control strategy may well be based on sensor signals to be discussed later.

In the following, the above noted process cycles will be explained in detail.

Preferably, as indicated in Fig. 1, the liquid network 6 comprises an inlet feed line 12 between the chamber inlet 3 and a cell broth source 13, preferably a storage vessel or production vessel such as a bioreactor. During a loading cycle, the cell broth may then be pumped by the liquid pumping arrangement 5 from the cell broth source 13 to the chamber inlet 3 via the inlet feed line 12, while the supernatant is flowing from the chamber outlet 4 to the filter arrangement 9 via the outlet supernatant line 10. In an especially preferred embodiment, during the loading cycle, the liquid pressure and/or the liquid flow at an inlet of the filter arrangement 9 may be controlled by the liquid pumping arrangement 5. This is due to the liquid tightness of the outlet supernatant line 10. It may also be necessary, that in the beginning of the loading cycle, remaining buffer in the centrifuge chamber 2.1 may be flushed into the waste reception 17 via the outlet waste line 14, before the chamber outlet 4 is connected by valve switching to the filter arrangement 9 via the outlet supernatant line 10.

Here it is to be understood, that the outlet supernatant line 10 and the outlet waste line 14 overlap each other along a certain liquid line section. Accordingly, the outlet supernatant line 10 on the one hand and the outlet waste line 14 on the other hand do not have to be separate from each other along their complete extent. This is true for all other definitions of liquid lines presented here, which are each being provided by part of the liquid network 6.

According to Fig. 2, the liquid network 6 preferably comprises an inlet washing line 15 between the chamber inlet 3 and a buffer source 16 and an outlet waste line 14 between the chamber outlet 4 and a waste reception 17, preferably a waste vessel, which here and preferably is the same as the waste reception 17. During a washing cycle, the buffer may be pumped by the liquid pumping arrangement 5 from the buffer source 16 to the chamber inlet 3 and from the chamber outlet 4 via the outlet supernatant line 10 to the filter arrangement 9 or from the chamber outlet 4 via the outlet waste line 14 to the waste reception 17. This means, that during the washing cycle, in an embodiment, the buffer may be pumped exclusively through the outlet supernatant line 10, and in another embodiment , the buffer may be pumped exclusively through the outlet waste line 14. In still another embodiment, during the washing cycle, the buffer may be pumped firstly through the outlet supernatant line 10 and subsequently through the outlet waste line 14.

Also during the washing cycle, the liquid pressure and/or the liquid flow in the outlet waste line 14 may be controlled by the liquid pumping arrangement 5. It may also be necessary, that in the beginning of the washing cycle, buffer and remaining supernatant in the centrifuge chamber 2.1 is being pumped to the filter arrangement 9 via the outlet supernatant line 10, before the chamber outlet 4 is connected by valve switching to the waste reception 17 via the outlet waste line 10.

Finally, it may be provided, that during the whole washing cycle, the chamber outlet 4 is connected by valve switching to the filter arrangement 9 via the outlet supernatant line 10. Then, the volume of the buffer to be pumped during the washing cycle is to be as low as possible, in order to prevent an unduly dilution of the supernatant in the supernatant reception 21.1. Preferably, during the washing cycle, buffer and remaining supernatant in the centrifuge chamber 2.1 is being pumped from the chamber outlet 4 via the outlet supernatant line 10 to the filter arrangement 9 for a short time, after which the washing cycle is being terminated.

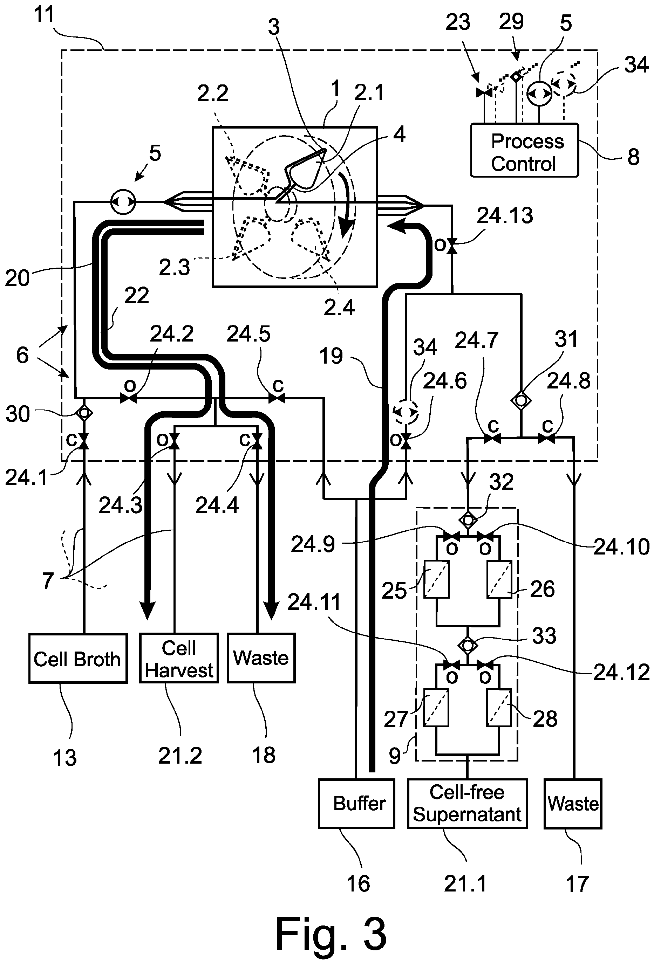

Fig. 3 shows, that the liquid network 6 comprises an outlet buffer line 19 between the chamber outlet 4 and the buffer source 16 and an inlet cell harvest line 20 between the chamber inlet 3 and a cell harvest reception 21.2, preferably a cell harvest vessel. This is for the case, that the cells in the cell broth are to be obtained as a product. During a discharging cycle, the buffer may then be pumped from the buffer source 16 to the chamber outlet 4 via the outlet buffer line 19, while the buffer including solid particles, preferably cell harvest, is flowing from the chamber inlet 3 to the cell harvest reception 21.2 via the inlet cell harvest line 20. During the discharging cycle, the liquid pressure and/or the liquid flow in the outlet buffer line 19 may be controlled by the liquid pumping arrangement 5.

In case, during discharging, the cells are to be dismissed, as an alternative to the inlet cell harvest line 20, there may be provided an inlet waste line 22 between the chamber inlet 3 and another waste reception 18, which may also be the waste reception 17 at the same time. In this case, during the discharging cycle, the buffer may be pumped from the buffer source 16 to the chamber outlet 4 via the outlet buffer line 19, while the buffer including solid particles, preferably cell harvest, is flowing from the chamber inlet 3 to the waste reception 18 via the inlet waste line 22.

The clarification setup preferably comprises a valve arrangement 23 with at least one valve 24, here and preferably with valves 24.1-24.13, controlled by the process control 8, that allows to deactivate and activate at least one of the liquid lines 7, preferably by closing and opening the respective valve 24. In the drawings, the valves 24.1-24.13 are assigned the status "c" for closed and "o" for opened. Accordingly, the valves 24 of the valve arrangement 8 are located within or at least at one end of the respective liquid lines 7 to be activated and deactivated.

For deactivation and activation of the respective liquid line 7, at least one valve 24 of the valve arrangement 23 is assigned to the respective liquid line 7. In the drawings, the valve arrangement 23 comprises valves 24.1 to 24.13 as noted above.

It is generally possible, that the filters 25-28 of the filter arrangement 9 are activated and deactivated manually, for example by manually controllable valves.
It is also possible, that no valve is assigned to the filter arrangement 9, in which case, instead of deactivating part of the filter arrangement, the flushing of the filter arrangement 9 is being started manually.

The term "flushing" means a pre-conditioning of filters by rinsing them with buffer before the centrifugation process is started. In Addition, after the centrifugation process is finished, a post-flushing of the filters is also prefered in some cases in order to flush out remaining product in the filters and filter lines into the harvest reception.

Here and preferably, however, it is provided that the valve arrangement 23 is controllable by the process control 8, such that activation and deactivation of the filters 25-28 of the filter arrangement 9 may be easily automated. The term "activate" and "deactivate" with regards to the filters 25-28 of the filter arrangement 9 generally means, that liquid flow through the respective filter 25-28 is enabled or disabled.

In the shown and insofar preferred embodiment the valve arrangement 23 allows to deactivate and activate at least one filter 25-28 of the filter arrangement 9, preferably by closing and opening the respective valve 24. Further preferably, for deactivation and activation of the respective filter 25-28, at least one valve 24 of the valve arrangement 23 is assigned to the respective filter 25-28.

The valve arrangement 23 also may allow to connect by valve switching the buffer source 16 to the filter arrangement 9 for flushing at least one filter 25-28 of the filter arrangement 9. For flushing, another liquid pumping arrangement 34, which is preferably assigned to the unit 11, may be provided as shown in the drawings.

Generally, the filter arrangement 9 may comprise only one filter stage. Here and preferably, though, the filter arrangement 9 comprises a first filter stage 25,26 and a subsequent second filter stage 27, 28, wherein, further preferably, the first filter stage 25, 26 comprises at least one depth filter and wherein the second filter stage 27, 28 comprises at least one sterile filter. In a particularly simple case, the first filter stage and the second filter stage comprise only one filter. Again, as an alternative, those filters may be activated and deactivated manually.

As noted above, the filter arrangement 9 may also comprise only a single filter stage. For example, the filter arrangement may then comprise a multi layer membrane filter, which combines the function of the depth filter with the function of the sterile filter.

Further preferably, the clarification setup comprises a sensor arrangement 29 for measuring properties of the liquid in at least one of the liquid lines 7, which sensor arrangement 29 provides sensor signals to the process control 8.

The process control 8 may activate or deactivate at least one of the liquid lines 7 via the valve arrangement 23 based on the sensor signals of the sensor arrangement 29 and according to the respective control strategy. It is preferred, that the sensor arrangement 29 comprises a biomass sensor arrangement 30, preferably an optical biomass sensor arrangement, for measuring an occurrence level of biomass, preferably the biomass concentration, in the respective liquid line 7. During the loading cycle, depending on the measured occurrence level, preferably biomass concentration, the process control 8 then deactivates or activates the inlet feed line 12 and/or starts the washing cycle.

As an alternative or in addition, the sensor arrangement 29 comprises a supernatant sensor arrangement 31, preferably an optical supernatant sensor arrangement, for measuring an occurrence level of supernatant in the respective liquid line 7. During the loading cycle and/or the washing cycle, depending on the measured occurrence level of supernatant, the process control 8 then deactivates or activates the outlet supernatant line 10 and the outlet waste line 14 via the valve arrangement 23.

Preferably during the loading cycle, the process control 8 activates or deactivates at least one filter 25-28 of the filter arrangement 9 based on the sensor signals of the sensor arrangement 29. In a particularly preferred embodiment, the sensor arrangement 29 comprises at least one pressure sensor 32, 33 assigned to a filter 25-28 of the filter arrangement 9, which pressure sensor 32, 33 measures the liquid pressure upstream of the respective filter 25-28. Further preferably, the process control 8 initiates an overpressure routine in case the measured liquid pressure exceeds a predefined value. It is also preferred, that the overpressure routine includes a flush routine of the respective filter 25-28 of the filter arrangement 9, and/or, that the overpressure routine includes circumpassing the respective filter 25-28 by a backup filter of the filter arrangement 9, and/or, that the overpressure routine includes terminating the loading cycle of the clarification setup.

Generally it may be provided, that the centrifuge chamber 2 is designed as a single use component. As an alternative or in addition, it may be advantageous, that at least part of the liquid network 6, in particular the outlet supernatant line 10, is designed as a single use component, further preferably, that at least part of the liquid filter arrangement 9 is designed as a single use component. The single use component, in particular the supernatant line 10, is preferably made of a plastic material at least partly, such that it may be realized with low effort. In a particularly preferred embodiment, the single use component is at least partly made of a silicon material and/or a polymer material.

In particular and as indicated above, the outlet supernatant line 10 as such may be provided as a single use component. With this, it is particularly easy to guarantee the proposed liquid tightness of the outlet supernatant line 10 without the risk of compromising the liquid tightness due to user errors during installation.

All in all it may be advantageous to provide all components, that are in direct contact with liquid, as single use components. This would at least include all liquid lines 7, all centrifuge chambers 2.1, 2.2, 2.3, 2.4, all filter 25-28 of the filter arrangement 9. It may also include at least part of the valve arrangement 23 and/or at least part of the sensor arrangement 29. In particular, single use sensors may be part of the sensor arrangement 29.

According to a separate teaching, the bioprocess installation with a proposed clarification setup and with a cell broth source 13 in the form of a storage vessel or production vessel, such as a bioreactor, is claimed as such. As noted above, all explanations given for the proposed clarification setup are fully applicable.

According to another separate teaching, a method for operating the proposed clarification setup is claimed as such, wherein the clarification setup comprises a valve arrangement 23 controlled by the process control 8 and wherein at least one filter 25-28 of the filter arrangement 9 is being deactivated and activated by closing and opening the respective valve 24 of the valve arrangement 23. As also noted above, all explanations given for the proposed clarification setup are fully applicable.

## Claims

1. Clarification setup of a bioprocessing installation for the clarification of a cell broth by centrifugation and subsequent filtration, wherein, for the centrifugation, the clarification setup comprises a centrifuge (1), wherein the centrifuge (1) comprises at least one centrifuge chamber (2.1) with a chamber inlet (3) and a chamber outlet (4), wherein the clarification setup comprises a liquid pumping arrangement (5) assigned to the centrifuge (1) and a liquid network (6) with a number of liquid lines (7) communicating with the liquid pumping arrangement (5), wherein the clarification setup comprises a process control (8) for controlling at least the centrifuge (1) and the liquid pumping arrangement (5),
**characterized in**
**that** for filtration, the clarification setup comprises a filter arrangement (9), that the liquid network (6) provides an outlet supernatant line (10) for a liquid connection between the chamber outlet (4) and the filter arrangement (9), which is at least temporarily, preferably permanently, liquid-tight, such that due to this liquid-tight condition of the outlet supernatant line (10) the liquid pumping arrangement (5) as such may drive the supernatant from the chamber outlet (4) to the filter arrangement (9) via the outlet supernatant line (10).

2. Clarification setup according to claim 1, **characterized in that** the centrifuge (1) and the liquid pumping arrangement (5) are combined in a preferably self-contained unit (11), preferably, that the filter arrangement (9) is part of this self-contained unit (11), and/or, that the process control (8) is part of this self-contained unit (11).

3. Clarification setup according to claim 1 or 2, **characterized in that** the process control (8) is designed to execute a number of predetermined process cycles sequentially by controlling at least the centrifuge (1) and the liquid pumping arrangement (5), preferably, that one of the process cycles is a loading cycle, during which cell broth is pumped into the chamber inlet (3), and/or, that one of the cycles is a washing cycle, during which a buffer is pumped into the chamber inlet (3), and/or, that one of the cycles is a discharging cycle, during which a buffer is pumped to the chamber outlet (4).

4. Clarification setup according to any one of the preceding claims, **characterized in that** the liquid network (6) comprises an inlet feed line (12) between the chamber inlet (3) and a cell broth source (13), preferably a storage vessel or production vessel, such as a bioreactor, and that during a loading cycle the cell broth may be pumped by the liquid pumping arrangement (5) from the cell broth source (13) to the chamber inlet (3) via the inlet feed line (12), while the supernatant is flowing from the chamber outlet (4) to the filter arrangement (9) via the outlet supernatant line (10), preferably, that during the loading cycle, the liquid pressure and/or the liquid flow at an inlet of the filter arrangement (9) may be controlled by the liquid pumping arrangement (5).

5. Clarification setup according to any one of the preceding claims, **characterized in that** the liquid network (6) comprises an inlet washing line (15) between the chamber inlet (3) and a buffer source (16) and an outlet waste line (14) between the chamber outlet (4) and a waste reception (17, 18), preferably a waste vessel, and that during a washing cycle the buffer may be pumped by the liquid pumping arrangement (5) from the buffer source (16) to the chamber inlet (3) and from the chamber outlet (4) via the outlet supernatant line (10) to the filter arrangement (9) or from the chamber outlet (4) via the outlet waste line (14) to the waste reception, preferably, that during the washing cycle, the liquid pressure and/or the liquid flow in the outlet waste line (14) may be controlled by the liquid pumping arrangement (5).

6. Clarification setup according to any one of the preceding claims, **characterized in that** the liquid network (6) comprises an outlet buffer line (19) between the chamber outlet (4) and a buffer source (16) and an inlet cell harvest line (20) between the chamber inlet (3) and a cell harvest reception (21.2), preferably a cell harvest vessel, and that during a discharging cycle the buffer may be pumped from the buffer source (16) to the chamber outlet (4) via the outlet buffer line (19), while the buffer including solid particles, preferably cell harvest, is flowing from the chamber inlet (3) to the cell harvest reception (21.2) via the inlet cell harvest line (20) or from the chamber inlet (3) to the waste reception (18) via the inlet waste line (22), preferably, that during the discharging cycle, the liquid pressure and/or the liquid flow in the outlet buffer line (19) may be controlled by the liquid pumping arrangement (5).

7. Clarification setup according to any one of the preceding claims, **characterized in that** the clarification setup comprises a valve arrangement (23) with valves (24), each controlled by the process control (8), which allows to deactivate and activate at least one of the liquid lines (7), preferably by closing and opening the respective valves (24) of the valve arrangement (23), further preferably, that, for deactivation and activation of the respective liquid line (7), at least one valve (24) of the valve arrangement (23) is assigned to the respective liquid line (7).

8. Clarification setup according to claim 7, **characterized in that** the valve arrangement (23) allows to deactivate and activate at least one filter of the filter arrangement (9), preferably by closing and opening the respective filter (25-28), further preferably, that, for deactivation and activation of the respective filter (25-28), at least one valve (24) of the valve arrangement (23) is assigned to the respective filter (25-28).

9. Clarification setup according to any one of the preceding claims, **characterized in that** the valve arrangement (23) allows to connect by valve switching the buffer source (16) to the filter arrangement (9) for flushing at least one filter (25-28) of the filter arrangement (9).

10. Clarification setup according to any one of the preceding claims, **characterized in that** the filter arrangement (9) comprises at least one filter stage, more preferably a first filter stage (25, 26) and a subsequent second filter stage (27, 28), preferably, that the first filter stage (25, 26) comprises at least one depth filter and that the second filter stage (27, 28) comprises at least one sterile filter.

11. Clarification setup according to any one of the preceding claims, **characterized in that** the clarification setup comprises a sensor arrangement (29) for measuring properties of the liquid in at least one of the liquid lines (7), which sensor arrangement (29) provides sensor signals to the process control (8).

12. Clarification setup according to any one of the preceding claims, **characterized in that** the process control (8) activates or deactivates at least one of the liquid lines (7) based on the sensor signals of the sensor arrangement (29), preferably, that the sensor arrangement (29) comprises a biomass sensor arrangement (30), preferably an optical biomass sensor arrangement, for measuring an occurrence level of biomass, preferably the biomass concentration, in the respective liquid line (7) and that during the loading cycle, depending on the measured occurrence level of biomass, the process control (8) deactivates/activates the inlet feed line (12), and/or, that the sensor arrangement (29) comprises a supernatant sensor arrangement (31), preferably an optical supernatant sensor arrangement, for measuring an occurrence level of supernatant in the respective liquid line (7) and that during the loading cycle and/or the washing cycle, depending on the measured occurrence level of supernatant, the process control (8) deactivates/activates the outlet supernatant line (10) and the outlet waste line (14).

13. Clarification setup according to any one of the preceding claims, **characterized in that**, preferably during the loading cycle, the process control (8) activates or deactivates at least one filter (25-28) of the filter arrangement (9) based on the sensor signals of the sensor arrangement (29), preferably, that the sensor arrangement (29) comprises at least one pressure sensor (32, 33) assigned to a filter (25-28) of the filter arrangement (9), which pressure sensor (32, 33) measures the liquid pressure upstream of the respective filter (25-28), further preferably, that the process control (8) initiates an overpressure routine in case the measured liquid pressure exceeds a predefined value, further preferably, that the overpressure routine includes a flush routine of the respective filter (25-28) of the filter arrangement (9), and/or, that the overpressure routine includes circumpassing the respective filter (25-28) by a backup filter of the filter arrangement (9), and/or, that the overpressure routine includes terminating the loading cycle of the clarification setup.

14. Clarification setup according to any one of the preceding claims, **characterized in that** the centrifuge chamber (2) is designed as a single use component, preferably, that at least part of the liquid network (6), in particular the outlet supernatant line (10), is designed as a single use component, further preferably, that at least part of the filter arrangement (9) is designed as a single use component.

15. Bioprocess installation with a clarification setup according to any one of the preceding claims and with a cell broth source (13) in the form of a storage vessel or a production vessel such as a bioreactor.

16. Method for operating a clarification setup according to any one of the claims 1 to 14 or a bioprocess installation according to claim 15, wherein the clarification setup comprises a valve arrangement (23) controlled by the process control (8) and wherein at least one filter (25-28) of the filter arrangement (9) is being deactivated and activated by closing and opening the respective valve (24) of the valve arrangement (23).

17. Method according to claim 16, **characterized in that** the clarification setup comprises a sensor arrangement (29) for measuring properties of the liquid in at least one of the liquid lines (7), which sensor arrangement (29) provides sensor signals to the process control (8) and that at least one filter (25-28) of the filter arrangement (9) is being deactivated/activated based on the sensor signals of the sensor arrangement (29).
